# NEUE EUROPÄISCHE PATENTSCHRIFT

(11) **EP 0 436 744 B2**
(45) Veröffentlichungstag und Bekanntmachung des Hinweises auf die Entscheidung über den Einspruch: **26.09.2001**
(45) Hinweis auf die Patenterteilung: 26.01.1994
(21) Anmeldenummer: 89120912.4
(22) Anmeldetag: 10.11.1989
(51) Int. Cl.: C07D 275/02, A01N 43/80, A01N 25/22

(54) **Stabilisierte wässrige Lösungen von 3-Isothiazolinonen**
Stabilized aqueous solutions of 3-Isothiazolinones
Solutions aqueuses stabilisées de 3-Isothiazolinones

(43) Veröffentlichungstag der Anmeldung: 17.07.1991
(73) Patentinhaber: THOR CHEMIE GMBH, D-67329 Speyer (DE)
(72) Erfinder: Schmidt, Hans-Jürgen, D-6720 Speyer (DE)
(74) Vertreter: DIEHL GLAESER HILTL & PARTNER

(56) Entgegenhaltungen:
- EP-A- 0 147 223
- EP-A- 0 315 464
- US-A- 3 975 155
- US-A- 4 067 878
- Seifen-öle-Fette-Wachse, 1987, 113. Jahrgang, Nr. 20, S. 776-779
- Schülke & Mayr Technisches Merkblatt "Parmetol K 40". 02.08.1982
- Rohm & Haas Technical Information "Kathon 886 F" Safety Guidelines, and European Product Safety Sheet, 1988

## Beschreibung

Die Erfindung betrifft stabilisierte wäßrige Lösungen eines oder mehrerer 3-Isothiazolinone gemäß dem Oberbegriff des Patentanspruchs 1 sowie deren Verwendung.

3-Isothiazolinone sind bekannte Verbindungen. Bei ihrer praktischen Verwendung, zum Beispiel als bakterizide, fungizide oder algizide Stoffe, haben sie den Nachteil, daß sie sich in Lösung relativ leicht zersetzen und damit unwirksam werden und gegebenenfalls auch unerwünschte Nebenprodukte bilden.

Es wurde bereits versucht, die Stabilität von 3-Isothiazolinonen durch Zusatz von beispielsweise Lösungsmitteln, Chloriden, Nitraten oder anderen Salzen, Formaldehyd, Formaldehyd bildenden Stoffen sowie Orthoestern oder anderen organischen Verbindungen zu verbessern. In der EP-A 0 315 464 sind stabilisierte, 3-Isothiazolone enthaltende Gemische beschrieben. Als Stabilisator dienen dort Orthoester. Diese sind aber sehr kostspielig.

Die EP-A 0 300 483 berichtet von 3-lsothiazolinonen, die durch Zusatz relativ komplex aufgebauter organischer Verbindungen stabilisiert werden. In dieser Druckschrift sind auch Isothiazolinonlösungen angegeben, die mit 1 % Kaliumpermanganat bzw. einer Kombination aus 1 % Kaliumpermanganat und 1 % Hydrochinon stabilisiert wurden. Im ersteren Fall handelt es sich nur um einen Vergleichsversuch, der ein unbefriedigendes Ergebnis bringt und nur zur Verdeutlichung dient, daß die alleinige Verwendung von Kaliumpermanganat zur Stabilisierung unzureichend ist. Im Falle der gleichzeitigen Anwesenheit von Hydrochinon, das bekanntlich ein starkes Reduktionsmittel darstellt, handelt es sich um einen gemäß der Druckschrift vorgeschlagenen Stabilisator. Daneben ist gemäß der Druckschrift auch Chinon als Oxidationsmittel eingesetzt worden. Dabei war aber gleichzeitig Magnesiumnitrat anwesend, mit dem das Chinon synergistisch zusammenwirkt. Die Druckschrift lehrt somit, daß die alleinige Verwendung eines Oxidationsmittels zur Stabilisierung des Isothiazolinons nicht genügt.

Aus der EP-A 0 147 223 sind Konservierungs- und Desinfektionsmittel bekannt, die als Wirkstoff beispielsweise ein Isothiazolinon und als Zusatzstoff ein organisches Hydroperoxid enthalten. Mit diesen bekannten Mitteln wird die Aufgabe gelöst, die Aktivität der Wirkstoffe, also zum Beispiel des Isothiazolinons, zu erhöhen. Die zugrundeliegende Aufgabe befaßt sich nicht mit der Stabilisierung der Wirkstoffe. Soweit dort Stabilisatoren genannt sind, geschieht dies nur beiläufig, wobei sich darunter keine Oxidationsmittel befinden.

In vielen Fällen der Anwendung von 3-Isothiazolinonen ist es aus verschiedenen Gründen wünschenswert, in den die 3-Isothiazolinone enthaltenden Bioziden den Gehalt der vorgenannten bekannten Stabilisatoren zu verringern. Beispielsweise ist auch bekannt, daß Dispersionen oder Emulsionen, die einen mikrobiziden Schutz benötigen, gegenüber Zusätzen von Salzen empfindlich sind. Dies gilt insbesondere dann, wenn die Salze zweiwertige lonen enthalten. Unterstützt durch eine schlechte Durchmischung entstehen dann leicht Koagulate, welche die Qualität der Dispersionen oder Emulsionen beeinträchtigen.

Ferner können die mikrobizid wirkenden Komponenten in diesen Koagulaten eingeschlossen werden. Dadurch stehen nur Teilmengen oder im ungünstigsten Fall keine mikrobizid wirkenden Stoffe zur Verfügung. Dies gilt insbesondere dann, wenn die zu konservierenden Produkte filtriert werden.

Diese unerwünschten Wirkungen der vorgenannten Bestandteile in Isothiazolinon enthaltenden Bioziden treten auch bei einigen Formulierungen auf, die mit salzarmen oder salzfreien, hydroxylgruppenhaltigen Lösungsmitteln stabilisiert worden sind.

Weiterhin gibt es Formulierungen, die keine oder nur eine geringe Konzentration an Chlorid enthalten dürfen, weil sonst eine störende Unverträglichkeit auftritt. Außerdem wird durch eine Verminderung des Chloridgehalts auch die Korrosionswirkung der Formulierung reduziert.

Bei Isothiazolinonformulierungen, die durch Nitrate stabilisiert wurden, besteht die Gefahr des Einschleppens oder der Bildung von Nitrosaminen. Letztere stehen im Verdacht, karzinogene Eigenschaften aufzuweisen. Deshalb besteht ein großes Bedürfnis dafür, Nitrate zur Stabilisierung zu vermeiden oder in ihrer Menge zu reduzieren. Im Falle von Formaldehyd und Formaldehyd abgebenden Stoffen als Stabilisatoren wurde zumindest in Tierversuchen nachgewiesen, daß Formaldehyd karzinogen wirkt.

Der Erfindung liegt die Aufgabe zugrunde, einen Weg zur Stabilisierung wäßriger Lösungen eines oder mehrerer Isothiazolinone anzugeben, der es ermöglicht, den bisher zur Stabilisierung nötigen Gehalt an Chlorid, Nitrat (und die damit verbundene unerwünschte Nitrosaminbildung), Nitrit, anderen Metallsalzen, Formaldehyd, Formaldehyd abgebenden Stoffen und/oder Lösungsmitteln (ausgenommen Wasser) zu vermindern oder ganz zu vermeiden.

Diese Aufgabe löst die Erfindung durch stabilisierte wäßrige Lösungen mit einem Gehalt an mindestens 0,1 Gew.-%, bezogen auf die gesamte Lösung, eines oder mehrerer 3-Isothiazolinone der allgemeinen Formel I oder II 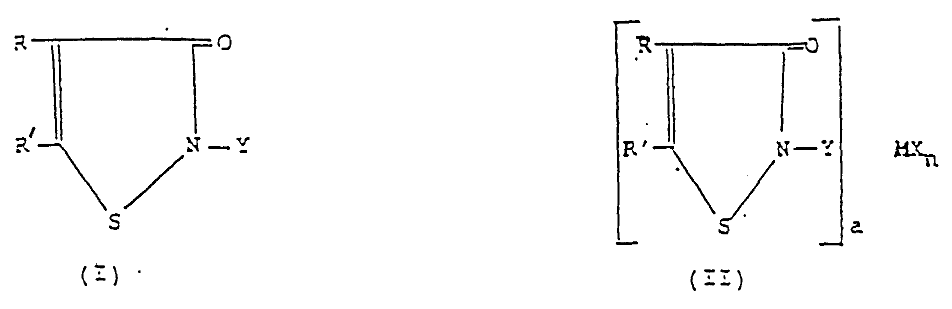 in denen
Y ein Wasserstoffatom oder einen unsubstituierten Alkylrest mit 1 bis 18 Kohlenstoffatomen, einen unsubstituierten oder halogen-substituierten Alkenyl- oder Alkinylrest mit 2 bis 8 Kohlenstoffatomen, einen Cycloalkylrest mit 5 bis 8 Kohlenstoffatomen, einen Aralkylrest mit 7 bis 11 Kohlenstoffatomen oder einen Arylrest mit 6 bis 10 Kohlenstoffatomen,
R und R' jeweils ein Wasserstoff- oder Halogenatom oder einen C₁ - bis C₄-Alkylrest.
M ein Kation in Form eines Metallkations, eines gegebenenfalls organische Reste tragenden Ammoniumions oder eines Pyridinium- oder Pyrimidiniumkations,
X ein mit dem Kalion M eine Verbindung bildendes Anion, a die Zahl 1 oder 2 und n eine ganze Zahl, die sich entsprechend der Wertigkeit des Kations M bei dessen Absättigung durch das Anion X ergibt, und mit einem Gehalt an 0,1 bis 3 Gew.-%, bezogen auf die gesamte Lösung, eines anorganischen Oxidationsmittels, ausgewählt aus Wasserstoffperoxid und Natriumperborat, dadurch gekennzeichnet, daß die Lösung frei von Metallnitrat und Kaliumpermanganat ist sowie gegebenenfalls 1 bis 20 ppm, bezogen auf die gesamte Lösung, eines Stabilistators für das Oxidationsmittel enthält.

Für das Kation M in Form eines Metallkations sind die Ionen von Barium. Blei, Cadmium, Calcium, Chrom, Eisen, Cobalt, Kupfer, Magnesium, Mangan, Nickel, Quecksilber, Silber, Strontium, Zinn und Zink bevorzugt.

Für das Anion X sind das Chlor-, Brom-, Jod-, Nitrit-, Sulfat-, Acetat-, Chlorat-, Perchlorat-. Bisulfat-, Bicarbonat-, Oxalat-, Maleat-, p-Toluolsulfonat-, Carbonat-und Phosphation bevorzugt.

Die erfindungsgemäßen Lösungen enthalten vorzugsweise 0,5 bis 3 Gew.-%, insbesondere 1,0 bis 2 Gew.-%, 3-Isothiazolinon, jeweils bezogen auf die gesamte Lösung.

Als Stabilisator für das Oxidationsmittel ist Diethylentriaminpentaessigsäure (DTPA) bevorz

Die Herstellung von 3-Isothiazolinonen ist in den Druckschriften US-3 523 121, US-3 517 022, US-3 761 488 und US-3 849 430 beschrieben. Die erfindungsgemäßen Lösungen enthalten als 3-Isothiazolinon vorzugsweise 5-Chlor-3-isothiazolinone, insbesondere 5-Chlor-2-methyl-3-isothiazolinon (CAS-Nr. 26 172-55-4). sowie 2-Methyl-3-isothiazolinon, 2-Octyl-3-isothiazolinon, 4,5-Dichlor-2-cyclohexyl-3-isothiazoiinon und/oder 4,5-Dichlor-2-octyl-3-isothiazolinon.

Das 5-Chlor-2-methyl-3-isothiazolinon wird in der erfindungsgemäßen Lösung vorzugsweise in einer Menge von 1,0 bis 1,5 Gew.-%, bezogen auf die gesamte Lösung, eingesetzt.

Bei der Herstellung von 5-Chlor-2-methyl-3-isothiazolinon entstehen als Nebenprodukte unchloriertes 2-Methyl-3-isothiazolinon der nachfolgenden Formel III und in sehr geringer Konzentration 4,5-Dichlor-2-methyl-3-isothiazolinon der nachfolgenden Formel IV oder deren Komplexe der nachfolgenden allgemeinen Formel V und VI 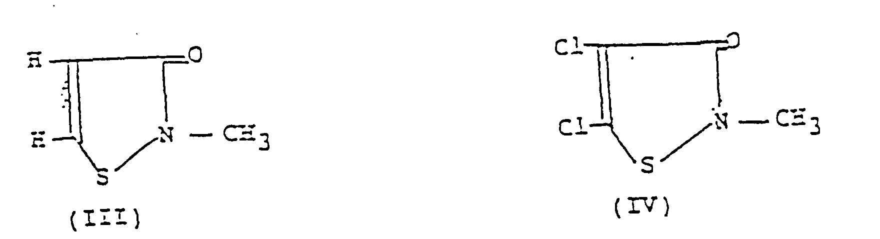 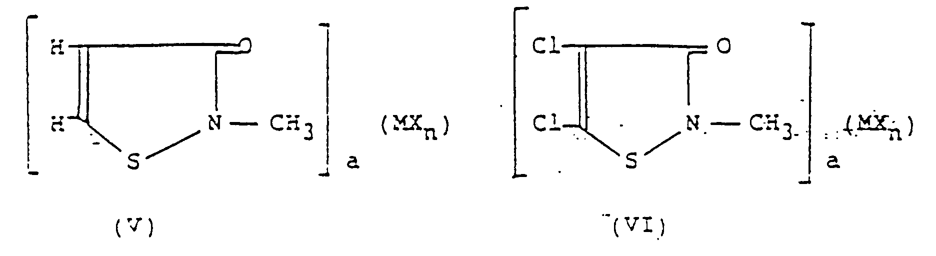 in denen M, X, n und a die vorstehenden Bedeutungen haben.

Gemäß einer speziellen Ausführungsform der Erfindung enthält die Lösung 1,0 bis 1,5 Gew.-% eines 5-Chlor-3-isothiazolinons, 0,3 Gew.-% Wasserstoffperoxid und 10 ppm Diethylentriaminpentaessigsäure, jeweils bezogen auf die gesamte Lösung.

Für die Verwendung der erfindungsgemäß zusammengesetzten Lösung hat es sich als zweckmäßig erwiesen, sie einer Wärmebehandlung bei einer Temperatur von 50 bis 100°C zu unterwerfen. Dies hat zum Beispiel den Vorteil, daß die zur Stabilisierung einzusetzende Wasserstoffperoxidkonzentration verringert werden kann.

Besonders bevorzugt ist die erfindungsgemäße Lösung, wenn sie frei von Chlorid, Nitrit, anderen Metallsalzen, Nitrosamin, Formaldehyd, Formaldehyd abgebenden Stoffen, Cu²⁺-Ionen und/oder Lösungsmitteln, ausgenommen Wasser, ist.

Für bestimmte Anwendungsfälle kann es aber zweckmäßig oder ausreichend sein, wenn die erfindungsgemäße Lösung noch Chlorid, Nitrit, ein anderes Metallsalz außer Nitrat, Formaldehyd, einen Formaldehyd abgebenden Stoff und/oder ein Lösungsmittel enthält. In solchen Fällen können aber diese bekannten Stabilisatoren in geringeren Konzentrationen verwendet werden als es ohne das erfindungsgemäß zugesetzte Oxidationsmittel möglich wäre.

Bei manchen 3-Isothiazolinonen ist es vorteilhaft, wenn die erfindungsgemäße Lösung zusätzlich einen die Löslichkeit des 3-Isothiazolinons in Wasser verbessernden Stoff enthält.

Die Herstellung der erfindungsgemäßen Lösungen erfolgt in üblicher Weise durch Auflösen der entsprechenden 3-Isothiazolinonhydrochloride in Wasser und anschließende Neutralisation mit Metallhydroxiden oder -oxiden.

Erfindungsgemäße Lösungen, die frei von anorganischen Neutralisationssalzen sind, werden durch Neutralisieren der 3-Isothiazolinonhydrochloride mit zum Beispiel organischen Basen, wie Trimethylamin, Triethylamin, Tripropylamin und cyclischen tertiären Aminen, beispielsweise Pyridin, erhalten.

Die erfindungsgemäßen Lösungen können zur Verbesserung ihrer bioziden Wirkung auch noch ein oder mehrere andere Biozide enthalten. Dazu gehören auch Biozide, die mit den 3-Isothiazolinonen eine synergistische Biozidkombination ergeben.

Die erfindungsgemäßen Lösungen werden vorzugsweise zur Verhinderung des Wachstums von Bakterien, Pilzen, Hefen oder Algen verwendet und können somit beispielsweise in folgenden Bereichen eingesetzt werden: Desinfektionsmittel (auch für den Bereich Medizin und für Krankenhäuser sowie Wäschereien), Sanitärreiniger, allgemeine Reinigungsmittel, Desodorantien, flüssige und pulverförmige Seifen, Öl- und Fettentferner, Molkereichemikalien, Holzschutzmittel, Farben, Lasuren, Beizen, Schimmelschutzmittel, Metallbearbeitungsflüssigkeiten, Kühlwasser, Luftwäscher, Erdölverarbeitung, Papierbehandlung, Papiermühlenschleimverhütungsmittel, Klebstoffe, Textilien (auch in ungewebter Form), Pigmentpasten, Latex, Gerbmittel, Lederbehandlungsmittel, Kraftstoffe, Mittel zur Anwendung in der Landwirtschaft und im Bergbau, Tinten, Lagerung von Erdöl, Mittel für Schwimmbäder, Kautschuk, Kosmetika, Toilettenartikel, Pharmazeutika, chemische Toiletten, Haushaltswaschmittel. Kraftstoffadditive, Schneidöle, Schutz- und Dekorfilme. Kunststoffe, Emulsionen, Wachse und Glanzmittel. Ganz allgemein eignen sich die erfindungsgemäßen Lösungen für eine Anwendung in Fällen, in denen Wasser und organische Materialien unter Bedingungen miteinander in Kontakt kommen, die ein unerwünschtes Wachstum von Mikroorganismen zulassen.

### Beispiel 1

Es wurde die Stabilität wäßriger Lösungen geprüft, die eine Kombination aus 5-Chlor-2-methyl-3-isothiazolinon und 2-Methyl-3-isothiazolinon in Verbindung mit verschiedenen Stabilisatoren enthielten. Dazu wurden die Lösungen bei erhöhten Temperaturen gelagert.

Es ist bekannt, daß nach folgendem Schema von erhöhten Lagertemperaturen auf die Lagerstabilitäten der Lösungen bei Normaltemperatur geschlossen werden kann. Eine Lagerzeit von einer Woche bei 50°C entspricht einer Lagerzeit von 2 Monaten bei Normaltemperatur, also 25°C. Eine Lagerzeit von 2 Wochen bei dieser höheren Temperatur entspricht einer Lagerzeit von 4 Monaten bei Normaltemperatur, eine Lagerzeit von 3 Wochen bei dieser höheren Temperatur entspricht einer Lagerzeit von 6 Monaten bei Normaltemperatur, eine Lagerzeit von 4 Wochen bei dieser höheren Temperatur entspricht einer Lagerzeit von 8 Monaten bei Normaltemperatur usw.

Eine Lagerung während einer Woche bei einer Temperatur von 65°C entspricht einer Lagerung von 7 Monaten bei Normaltemperatur, eine Lagerung von 2 Wochen bei dieser höheren Temperatur entspricht einer Lagerung von 14 Monaten bei Normaltemperatur, eine Lagerung von 3 Wochen bei dieser höheren Temperatur entspricht einer Lagerung von 21 Monaten bei Normaltemperatur usw.

Es wurde eine wäßrige Lösung mit einem Gehalt von 1,5 Gew.-% eines Gemisches aus 5-Chlor-2-methyl-3-isothiazolinon und 2-Methyl-3-isothiazolinon (Gew.-Verhältnis 3 : 1) hergestellt. Dazu wurden die entsprechenden Isothiazolinonhydrochloride in wäßriger Lösung mit verdünnter Kaliumhydroxidlösung bis zu einem pH-wert von 3,5 neutralisiert. Anschließend wurde der jeweilige Stabilisator zugesetzt, wie er in den nachfolgenden Tabellen I, II und III angegeben ist.

Die erhaltenen Lösungen wurden dem vorgenannten Lagertest bei Temperaturen von 50 und 65°C unterworfen und in dieser Zeit nach den unten angegebenen Zeiträumen mittels Hochleistungsflüssigchromatographie (HPLC) auf ihren restlichen Isothiazolinongehalt überprüft. Bei einer Restmenge von mindestens 85 Gew.-% des eingesetzten 3-Isothiazolinons wurde die Lösung als ausreichend stabil beurteilt.

In den Tabellen I, II und III haben die Symbole folgende Bedeutungen:
+ bedeutet > 85 % des eingesetzten 3-Isothiazolinons
0 bedeutet > 50 % < 85 % des eingesetzten 3-Isothiazolinons
- bedeutet < 50 % des eingesetzten 3-Isothiazolinons
In der Tabelle I sind durch die Symbole die Restmengen des eingesetzten 3-Isothiazolinons angegeben, die bei einer Lagertemperatur von 50 °C gemessen wurden. Aus den Tabellen II und III sind die entsprechenden Ergebnisse bei einer Lagertemperatur von 65°C ersichtlich.

In den Tabellen beziehen sich die Prozentangaben und die Mengen in "ppm" jeweils auf das Gewicht der gesamten Lösung.

**Tabelle I**

| Stabilisator | Lagerzeit, Wochen | | | | | | | | |
|---|---|---|---|---|---|---|---|---|---|
| | 1 | 2 | 3 | 4 | 5 | 6 | 8 | 10 | 12 |
| ohne | - | - | - | - | - | - | - | - - | |
| 0,3% Wasserstoffperoxid | + | + | + | + | + | 0 | - | - | - |
| 0,3% Wasserstoffperoxid und 10 ppm DTPA | + | + | + | + | + | + | + | + | + |
| 0,5% Wasserstoffperoxid und 1,5% Magnesiumnitrat | + | + | + | + | + | + | + | + | - |
| 2% Natriumperborat | + | + | + | + | + | + | 0 | - | - |
| 2% Natriumperborat und 0,1% DTPA | + | + | + | + | + | + | 0 | 0 | - |

**Tabelle II**

| Stabilisator | Lagerzeit, Tage | | | | | | |
|---|---|---|---|---|---|---|---|
| | 2 | 4 | 6 | 8 | 10 | 12 | 14 |
| ohne | - | - | - | - | - | - | - |
| 0,3 % Wasserstoffperoxid | + | + | 0 | - | - | - | - |
| 0,3 % Wasserstoffperoxid und 10 ppm DTPA | + | + | + | + | + | + | 0 |
| 2 % Natriumperborat | + | + | + | + | 0 | - | - |
| 2 % Natriumperborat und 0,1 % DTPA | + | + | + | + | + | 0 | - |
| 0,3 % Wasserstoffperoxid und 0,05 % Phosphorsäure | + | + | + | 0 | - | - | - |
| 1,5 % Magnesiumnitrat | - | - | - | - | - | - | - |

Entsprechend den vorgenannten Versuchen wurden auch die Stabilitäten von Lösungen untersucht, die 1,5 Gew.-% eines chloridfreien Gemisches aus 5-Chlor-2-methyl-3-isothiazolinon und 2-Methyl-3-isothiazolinon (Gewichtsverhältnis 3 : 1) enthielten. Diese Lösungen wurden mit den in der Tabelle III angegebenen Stabilisatoren versetzt. Die Ergebnisse sind in der Tabelle III zusammengefaßt.

**Tabelle III**

| Stabilisator | Lagerzeit, Tage | | | | | |
|---|---|---|---|---|---|---|
| | 1 | 3 | 5 | 10 | 12 | 15 |
| ohne | - | - | - | - | - | - |
| 0,3 % Wasserstoffperoxid und 10 ppm DTPA | + | + | + | + | + | 0 |
| 1,5 % Magnesiumnitrat | - | - | - | - | - | - |
| 2,0 % Kaliumnitrat | - | - | - | - | - | - |
| 1,2 % Magnesiumsulfat | - | - | - | - | - | - |
| 1.2 % Magnesiumsulfat und 0,3 % Wasserstoffperoxid und 10 ppm DTPA | + | + | + | + | + | 0 |

Aus den Tabellen I, II und III ist ersichtlich, daß die erfindungsgemäß eingesetzten Stabilisatoren Wasserstoffperoxid und Natriumperborat den bekannten Stabilisatoren Magnesiumnitrat, Kaliumnitrat und Magnesiumsulfat überlegen sind, was vor allem für die Lagerung bei einer Temperatur von 65°C gilt.

### Beispiel 2

Eine erfindungsgemäße Lösung wurde mit in bekannter Weise durch Nitrate stabilisierte 3-Isothiazolinonlösungen hinsichtlich der minimalen Hemmkonzentration für Bakterien nach der Boillon-Verdünnungsmethode gemäß DIN 58 940, Teil 5, verglichen. Es wurden wäßrige Lösungen eines Gemisches aus 5-Chlor-2-methyl-3-isothiazolinon und 2-Methyl-3-isothiazolinon (Gewichtsverhältnis 3 : 1) eingesetzt. Die jeweils verwendeten Stabilisatoren, Stoffmengen und Bakterien sind in der nachfolgenden Tabelle IV angegeben.

**Tabelle IV**

| Probe | Gehalt an 3-Isothiazolinon in der Lösung, % | Gehalt an Stabilisator | Minimale Hemmkonzentration*, ppm 3-Isothiazolinon | | | |
|---|---|---|---|---|---|---|
| | | | Pr.vul. | Ps.ae. | Kl.ae. | Esch.c. |
| 1 | 1,5 | 4% Mg (NO₃)₂ | 1 | 1 | 1 | 1 |
| 2 | 1,5 | 1,5% Mg (NO₃)₂ und 0,15% Cu(NO₃)₂ | 0,5 | 0,5 | 0,5 | 0,5 |
| 3 | 1,5 | 0,3% H₂O₂ und 10 ppm DTPA | 0,5 | 0,5 | 0,5 | 0,5 |

| | | | | | | |
|---|---|---|---|---|---|---|
| * Pr. vul = Proteus vulgaris Keimzahl: 2,2 · 10⁶ Ps. ae. = Pseudomonas aeruginosa Keimzahl: 2,4 · 10⁶ Kl. ae. = Klebsiella aerogenes Keimzahl: 8,0 · 10⁶ Esch. c. = Escherichia coli Keimzahl: 2,2 · 10⁶ | | | | | | |

Aus der Tabelle IV ist ersichtlich, daß die minimale Hemmkonzentration der erfindungsgemäßen Lösung der minimalen Hemmkonzentration von in bekannter Weise stabilisierten Lösungen entspricht. Das bedeutet, daß das erfindungsgemäß als Stabilisator eingesetzte Oxidationsmittel die minimale Hemmkonzentration nicht beeinträchtigt, gleichzeitig aber die Möglichkeit schafft, unerwünschte Nitrate als Stabilisatoren zu vermeiden

### Beispiel 3

Eine erfindungsgemäße Lösung wurde mit in bekannter Weise stabilisierten Lösungen von 3-Isothiazolinonen hinsichtlich der Wirksamkeit gegenüber Pilzen und Hefen mit dem AgarDiffusionstest nach DIN 58 940, Teil 3, verglichen. Bei den drei nachfolgend angegebenen Formulierungen 1, 2 und 3 wurde jeweils eine wäßrige Lösung eines Gemisches aus 5-Chlor-2-methyl-3-isothiazolinon und 2-Methyl-3-isothiazolinon (Gewichtsverhältnis 3 : 1) eingesetzt.

### Formulierung 1:

Gehalt an 3-Isothiazolinon 1,5 %
Stabilisiert mit 4 % Mg (NO₃)₂
Neutralisationssalz 0,9 % MgCl₂

### Formulierung 2:

Gehalt an 3-Isothiazolinon 1,5 %
Stabilisiert mit 1,5 % Mg (NO₃)₂ und 0,15 % Cu (NO₃)₂
Neutralisationssalz 0,9 % MgCl₂

### Formulierung 3:

Gehalt an 3-Isothiazolinon 1,5 %
Stabilisiert mit 0,3 % H₂O₂ und 10 ppm DTPA
Neutralisationssalz 1,15 % KCI
Die Ergebnisse sind in den nachfolgenden Tabellen V und VI zusammengefaßt.

**Tabelle V**

| Formulierung | 3-Isothiazolinon- konzentration, ppm | Inhibitationszone auf Malzagar, mm | | | | |
|---|---|---|---|---|---|---|
| | | Species* | | | | |
| | | Asp.nig. | Fus.sp. | Pen.fun. | Sac.cer. | Rho.rub. |
| 1 | 15 | 2 | 0 | 4 | 2 | 1 |
| | 22,5 | 3 | 1 | 5 | 3 | 2 |
| | 30 | 3 | 2 | 6 | 3 | 3 |
| 2 | 15 | 1 | 0,5 | 5 | 1 | 2 |
| | 22,5 | 2 | 2 | 6 | 2 | 3 |
| | 30 | 3 | 3 | 7 | 3 | 4 |
| 3 | 15 | 2 | 1 | 5 | 2 | 2 |
| | 22,5 | 3 | 2 | 6 | 3 | 3 |
| | 30 | 3 | 3 | 7 | 4 | 4 |

| | | | | | | |
|---|---|---|---|---|---|---|
| * Asp.nig. = Aspergillus niger Fus.sp. = Fusarium sp. Pen.fun. = Penicillium funiculosum Sac.cer. = Saccharomyces cerevisiae Rho.rub. = Rhodotorula rubra | | | | | | |

**Tabelle VI**

| Formulierung | 3-Isothiazolinonkonzentration, ppm | Inhibitionszone auf Kartoffeldextroseagar, mm | | | | |
|---|---|---|---|---|---|---|
| | | Species* | | | | |
| | | Asp.nig. | Fus.sp. | Pen.fun. | Sac.cer. | Rho.rub. |
| 1 | 15 | 1 | 0,5 | 4 | 2 | 1 |
| | 22,5 | 2 | 2 | 5 | 3 | 2 |
| | 30 | 3 | 3 | 6 | 3 | 3 |
| 2 | 15 | 2 | 1 | 4 | 1 | 2 |
| | 22,5 | 3 | 2 | 5 | 2 | 3 |
| | 30 | 3 | 4 | 6 | 3 | 4 |
| 3 | 15 | 1 | 1 | 4 | 1 | 2 |
| | 22,5 | 2 | 2 | 5 | 2 | 3 |
| | 30 | 3 | 2 | 6 | 3 | 4 |

| | | | | | | |
|---|---|---|---|---|---|---|
| * Siehe Tabelle V | | | | | | |

Aus den Tabellen V und VI ist ersichtlich, daß die erfindungsgemäßen Lösungen eine mindestens ebenso große Wirksamkeit aufweisen wie die in bekannter Weise stabilisierten Lösungen.

### Beispiel 4

Es wurde die Wirkung einer erfindungsgemäßen Lösung auf eine 30-prozentige Reinacrylatemulsion untersucht. Im Vergleich dazu wurden in bekannter Weise stabilisierte 3-Isothiazolinonlösungen geprüft. Die in der nachfolgenden Tabelle VII angegebenen Formulierungen 1, 2 und 3 hatten die gleiche Zusammensetzung wie jene im Beispiel 3.

Beim Versetzen von Polymeremulsionen mit Konservierungsmitteln in Form von Isothiazolinonen, die Neutralisationssalze mit zweiwertigen Metallionen, wie Mg²⁺ und Cu²⁺ enthalten, kann ein sogenannter "Salzschock" eintreten, d.h. es bildet sich ein Bodensatz oder eine gelatineartige Masse. Dies ist natürlich unerwünscht.

Für den vorgenannten Vergleich wurde zunächst die Polyacrylatemulsion durch ein Sieb (325 Maschen) geführt, um ein bei der Herstellung der Emulsion eventuell entstandenes Gel zu entfernen.

Anschließend wurde jeweils eine Probe der Emulsion mit der genannten Formulierung 1, 2 oder 3 versetzt, wobei eine Konzentration von 37,5 ppm 3-Isothiazolinon, bezogen auf das gesamte Gemisch, eingestellt wurde. Die 3-Isothiazolinonlösung wurde in 100 g der Emulsion gegeben, die sich in einer 100 ml-Flasche mit Schraubverschluß befand. Der Ansatz wurde gut verrührt und eine Stunde bei Raumtemperatur gelagert. Anschließend wurde der Ansatz durch ein Sieb (325 Maschen) geführt. Der abgesiebte Rückstand wurde mit deionisiertem Wasser gewaschen, um nichtkoagulierte Emulsion daraus zu entfernen. Der Siebrückstand wurde gesammelt und über Nacht bei einer Temperatur von 50°C sowie anschließend während einer Stunde bei 150°C getrocknet.

Die Ergebnisse sind in der nachfolgenden Tabelle Vll angegeben.

**Tabelle VII**

| Formulierung | Siebrückstand, mg/kg Emulsion |
|---|---|
| 1 | 2072 |
| 2 | 933 |
| 3 | kein Rückstand |

## Patentansprüche

1. Stabilisierte wäßrige Lösung mit einem Gehalt an mindestens 0,1 Gew.-%, bezogen auf die gesamte Lösung, eines oder mehrerer 3-Isothiazolinone der allgemeinen Formel I oder II 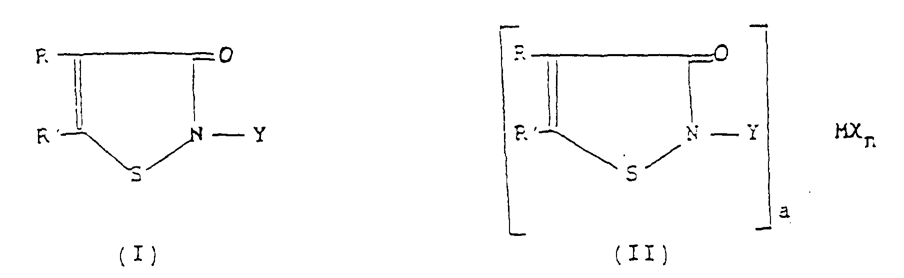 in denen
Y ein Wasserstoffatom oder einen unsubstituierten Alkylrest mit 1 bis 18 Kohlenstoffatomen, einen unsubstituierten oder halogensubstituierten Alkenyl- oder Alkinylrest mit 2 bis 8 Kohlenstoffatomen, einen Cycloalkylrest mit 5 bis 8 Kohlenstoffatomen, einen Aralkylrest mit 7 bis 11 Kohlenstoffatomen oder einen Arylrest mit 6 bis 10 Kohlenstoffatomen,
R und R' jeweils ein Wasserstoff- oder Halogenatom oder einen C₁- bis C₄-Alkylrest,
M ein Kation in Form eines Metallkations, eines gegebenenfalls organische Reste tragenden Ammoniumions oder eines Pyridinium- oder Pyrimidiniumkations,
X ein mit dem Kation M eine Verbindung bildendes Anion,
a die Zahl 1 oder 2 und n eine ganze Zahl, die sich entsprechend der Wertigkeit des Kations M bei dessen Absättigung durch das Anion X ergibt,
bedeuten,
und mit einem Gehalt an 0,1 bis 3 Gew.-%, bezogen auf die gesamte Lösung, eines anorganischen Oxidationsmittels, ausgewählt aus Wasserstoffperoxid und Natriumperborat,
**dadurch gekennzeichnet, daß** die Lösung frei von Metallnitrat und Kaliumpermanganat ist
sowie gegebenenfalls 1 bis 20 ppm, bezogen auf die gesamte Lösung, eines Stabilisators für das Oxidationsmittel enthält.

2. Lösung nach Anspruch 1, **dadurch gekennzeichnet, daß** sie als Stabilisator für das Oxidationsmittel Diethylentriaminpentaessigsäure enthält.

3. Lösung nach Anspruch 1 oder 2, **dadurch gekennzeichnet, daß** sie als 3-Isothiazolinon das 5-Chlor-2-methyl-3-isothiazolinon, 2-Methyl-3-isothiazolinon, 2-Octyl-3-isothiazolinon, 4,5-Dichlor-2-cyclohexyl-3-isothiazolinon und/oder 4,5-Dichlor-2-octyl-3-isothiazolinon enthält.

4. Lösung nach Anspruch 1, **dadurch gekennzeichnet, daß** sie 1,0 bis 1,5 Gew.-% eines 5-Chlor-3-isothiazolinons, 0,3 Gew.-% Wasserstoffperoxid und 10 ppm Diethylentriaminpentaessigsäure, jeweils bezogen auf die gesamte Lösung, enthält.

5. Lösung nach einem der Ansprüche 1 bis 4, **dadurch gekennzeichnet, daß** sie einer Wärmebehandlung bei einer Temperatur von 50 bis 100°C unterworfen worden ist.

6. Lösung nach einem der Ansprüche 1 bis 5, **dadurch gekennzeichnet, daß** sie frei von Chlorid, Nitrit, anderen Metallsalzen, Nitrosamin, Formaldehyd, Formaldehyd abgebenden Stoffen, Cu²⁺-Ionen und/oder Lösungsmitteln, ausgenommen Wasser, ist.

7. Lösung nach einem der Ansprüche 1 bis 5, **dadurch gekennzeichnet, daß** sie Chlorid, Nitrit, ein anderes Metallsalz außer ein Metallnitrat, Formaldehyd, einen Formaldehyd abgebenden Stoff und/oder ein Lösungsmittel enthält.

8. Lösung nach einem der Ansprüche 1 bis 7, **dadurch gekennzeichnet, daß** sie einen die Löslichkeit des 3-Isothiazolinons in Wasser verbessernden Stoff enthält.

9. Verwendung einer Lösung nach einem der Ansprüche 1 bis 8 zur Verhinderung des Wachstums von Bakterien, Pilzen, Hefen oder Algen.

## Claims

1. Stabilised aqueous solution with a content of at least 0.1 wt.%, taken on the entire solution of one or more 3-isothiazolinones of the general formula I or II 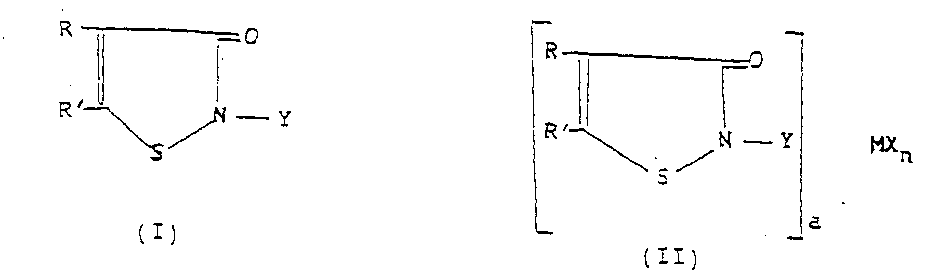 in which
Y represents a hydrogen atom or an unsubstituted alkyl group with 1 to 18 carbon atoms, an unsubstituted or halo substituted alkenyl or alkinyl group with 2 to 8 carbon atoms, a cycloalkyl group with 5 to 8 carbon atoms, an aralkyl group with 7 to 11 carbon atoms or an aryl group with 6 to 10 carbon atoms,
R and R' in each case a hydrogen or halogen atom or a C₁-to C₄-alkyl group,
M a cation in the form of a metal cation, an optionally organic group carrying ammonium ion or a pyridinium or pyrimidinium cation,
X an anion forming a compound with the cation M,
a the number 1 or 2 and n a whole number which results from the valence of the cation M on its saturation by the anion X,
and having a content of 0.1 to 3 wt.%, taken on the entire solution of an inorganic oxidising agent selected from hydrogen peroxide and sodium perborate,
**characterized in that** the solution is free of metal nitrate and potassium permanganate,
as well as optionally contains 1 to 20 ppm taken on the entire solution of a stabilizer for the oxidising agent.

2. Solution according to Claim 1, **characterised in that** it contains diethylenetriamine penta-acetic acid as stabiliser for the oxidising agent.

3. Solution according to Claims 1 or 2, **characterised in that** it contains as 3-isothiazolinone the 5-chloro-2-methyl-3-isothiazolinone, 2-methyl-3-isothiazolinone, 2-octyl-3-isothiazolinone, 4,5-dichloro-2-cyclohexyl-3-isothiazolinone and/or 4,5-dichloro-2-octyl-3-isothiazolinone.

4. Solution according to Claim 1, **characterised in that** it contains 1.0 to 1.5 Wt.% of a 5-chloro-3-isothiazolinone, 0.3 wt.% hydrogen peroxide and 10 ppm diethylenetriamine penta-acetic acid in each case taken on the entire solution.

5. Solution according to one of Claims 1 to 4, **characterised in that** it has been subjected to a heat treatment at a temperature of 50 to 100 °C.

6. Solution according to one of Claims 1 to 5, **characterised in that** it is free of chloride, nitrite, other metal salts, nitrosamine, formaldehyde, formaldehyde providing materials, Cu²⁺ ions and/or solvents with the exception of water.

7. Solution according to one of Claims 1 to 5, **characterised in that** it contains chloride, nitrite, another metal salt with the exception of a metal nitrate, formaldehyde, a formaldehyde providing material and/or a solvent.

8. Solution according to one of Claims 1 to 7, **characterised in that** it contains a material improving the solubility of the 3-isothiazolinone in water.

9. The use of a solution according to one of Claims 1 to 8 to prevent the growth of bacteria, fungi, yeasts or algae.

## Revendications

1. Solution aqueuse stabilisée contenant au moins 0,1 % en poids, par rapport à la solution totale, d'une ou plusieurs 3-isothiazolinones ayant les formules générales I ou II 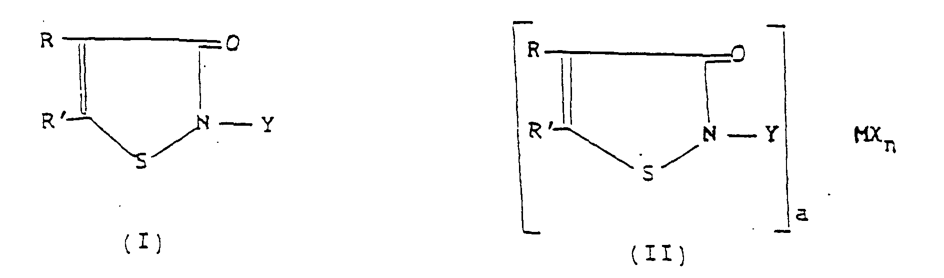 dans laquelle
Y est un atome d'hydrogène ou un radical alkyle non substitué ayant de 1 à 18 atomes de carbone, un radical alcényle ou alcynyle non substitué ou halogéné ayant de 2 à 8 atomes de carbone, un radical cycloalkyle ayant de 5 à 8 atomes de carbone, un radical aralkyle ayant de 7 à 11 atomes de carbone ou un radical aryle ayant de 6 à 10 atomes de carbone,
les radicaux R et R' sont chacun un atome d'hydrogène ou d'halogène ou un radical alkyle en C₁ à C₄.
M est un cation sous forme d'un cation métallique, d'un ion ammonium portant éventuellement des résidus organiques, ou d'un cation pyridinium ou pyrimidinium,
X est un anion formant un composé avec le cation M,
a est le nombre 1 ou 2, et n est un nombre entier qui correspond à la valeur du cation M quand il est saturé par l'anion X,
et ayant un contenu de 0,1 à 3 % en poids, par rapport à la solution totale, d'un oxydant minéral, sélectionné d'un peroxide d'hydrogène et de perborate de sodium,
**caractérisé en ce que** la solution est exempte de nitrate de métal et de permanganate de potassium
ainsi qu'elle contient optionellement de 1 à 20 ppm par rapport à la solution totale d'un stabilisant de l'oxydant.

2. Solution selon la revendication 1, **caractérisée en ce qu'**elle contient comme stabilisant de l'oxydant de l'acide diéthylènetriaminepentaacétique.

3. Solution selon la revendication 1 ou 2, **caractérisée en ce qu'**elle contient en tant que 3-isothiazolinone la 5-chloro-2-méthyl-3-isothiazolinone, la 2-méthyl-3-isothiazolinone, la 2-octyl-3-isothiazolinone, la 4,5-dichloro-2-cyclohexyl-3-isothiazolinone et/ou la 4,5-dichloro-2-octyl-3-isothiazolinone.

4. Solution selon la revendication 1, **caractérisée en ce qu'**elle contient de 1,0 à 1,5 % en poids d'une 5-chloro-3-isothiazolinone, 0,3 % en poids de peroxyde d'hydrogène et 10 ppm d'acide diéthylènetriaminepentaacétique, toujours par rapport à la solution totale.

5. Solution selon l'une des revendications 1 à 4, **caractérisée en ce qu'**elle a été soumise à un traitement thermique à une température de 50 à 100 °C.

6. Solution selon l'une des revendications 1 à 5, **caractérisée en ce qu'**elle est exempte de chlorure, de nitrite, d'autres sels métalliques, de nitrosamine, de formaldéhyde, de substances produisant du formaldéhyde, d'ions Cu²⁺ et/ou de solvants, à l'exception de l'eau.

7. Solution selon l'une des revendications 1 à 5, **caractérisée en ce qu'**elle contient du chlorure, du nitrite, un autre sel métallique à l'exception d'un nitrate de métal, du formaldéhyde, une substance produisant du formaldéhyde et/ou un solvant.

8. Solution selon l'une des revendications 1 à 7, **caractérisée en ce qu'**elle contient une substance améliorant la solubilité de la 3-isothiazolinone dans l'eau.

9. Utilisation d'une solution selon l'une des revendications 1 à 8 pour inhiber la croissance de bactéries, de champignons, de levures ou d'algues.
